# EUROPEAN PATENT SPECIFICATION

(11) **EP 3 849 652 B1**
(45) Date of publication and mention of the grant of the patent: **10.01.2024**
(21) Application number: 19755929.7
(22) Date of filing: 23.08.2019
(51) Int. Cl.: A61M 39/02, A61M 39/12, F16L 33/22

(54) **PORT FOR A CATHETER FOR SUBCUTANEOUS IMPLANTATION INTO A PATIENT**
ANSCHLUSS FÜR KATHETER ZUR SUBKUTANEN IMPLANTATION IN EINEN PATIENTEN
ORIFICE D'UN CATHÉTER POUR UNE IMPLANTATION SOUS-CUTANÉE CHEZ UN PATIENT

(30) Priority: 12.09.2018 EP 18194042
(43) Date of publication of application: 21.07.2021
(73) Proprietor: Fresenius Kabi Deutschland GmbH, 61352 Bad Homburg (DE)
(72) Inventor: HUTH, Matthias, 99831 Ifta (DE); PAPIOREK, Martina, 65597 Hünfelden 1 (DE)
(74) Representative: Fresenius Kabi Deutschland GmbH
(86) International application number: PCT/EP2019/072554
(87) International publication number: WO 2020/052940

(56) References cited:
- WO-A2-96/37254
- US-A- 5 178 612
- US-A1- 2006 264 911
- US-A1- 2014 236 105

## Description

The invention relates to a port for a catheter for subcutaneous implantation into a patient according to claim 1.

A port of this kind comprises a housing, a cavity arranged in the housing for receiving a fluid, a needle-penetrable closure element placed on the housing and penetrable by a puncturing needle for accessing the cavity, a connector for connecting the catheter to the port, and a fixing element for fixing the catheter on the connector. The connector comprises a shaft portion for receiving the catheter and a flow channel formed in the shaft portion and being in fluid connection with the cavity.

A port of this kind, as it is known for example from US 5 178 612 A and EP 1 675 641 B1, can be implanted into a patient in that it for example is inserted subcutaneously beneath the skin of a patient. The port serves for infusing a medical drug, a blood product, a nutritional fluid or another medical fluid into the venous or arterial system of a patient. By means of the port a medical fluid can be given to a patient in a repeated fashion over a rather long period of time. Because the port is completely implanted under the skin of a patient, the risk for infections is reduced, and a medical fluid can be administered to the patient for treatment over a long period of time without the patient having to be stationary in a hospital and without the port impacting the everyday life of the patient.

During an infusion, a medical fluid is guided from the cavity enclosed in the housing via the connector to a catheter connected to the connector and via the catheter to a location of action in the patient, for example into the venous or arterial system of the patient. The catheter herein together with the port is implanted into the patient and is placed in the patient such that the medical fluid can be transported to the predefined location of action in the patient.

The assembly of the port, i.e., the connection of the catheter to the port, generally takes place during surgery and requires a surgeon to place the catheter on the connector of the port and to fix the catheter on the connector by means of a suitable fixing element. In this regard it is desirable to provide a port which allows for an easy handling for connecting a catheter to the port in order to facilitate the assembly of the port and the placement of a catheter on the port in particular for a surgeon during surgery.

EP 2 603 253 B1 discloses a locking apparatus for locking a catheter on an implantable vascular access port. The locking apparatus comprises a collet sleeve and a lockable insert. The collet sleeve and the lockable insert, which both are separate from the port, are movable with respect to each other in order to clamp a catheter to a stem provided on the port.

It is an object of the instant invention to provide a port for a catheter which allows for an easy handling in particular for connecting a catheter to the port.

This object is achieved by means of a port comprising the features of claim 1.

Accordingly, the fixing element comprises a body portion, a clamping element protruding from the body portion along a fixing direction and at least one locking element formed on the body portion. In a preassembled state of the fixing element the at least one locking element is in positive locking engagement with a first counter-locking element formed on the housing to hold the fixing element on the housing in the preassembled state. In an assembled state of the fixing element the at least one locking element is in positive locking engagement with a second counter-locking element formed on the housing to fix the fixing element with respect to the housing in the assembled state. The fixing element herein is movable with respect to the housing along the fixing direction for transferring the fixing element from the preassembled state into the assembled state, the clamping element being configured to act onto the catheter in the assembled state of the fixing element to fix the catheter on the shaft portion of the connector.

The fixing element hence is preassembled on the housing of the port already prior to placing the catheter on the corresponding connector of the port. Because the fixing element is preassembled on the housing, the fixing element cannot be lost prior to assembly of the catheter. In addition, during assembly the fixing element does not need to be placed on the housing, but simply can be transferred from the preassembled state to the assembled state in order to fix the catheter on the connector.

In the preassembled state the fixing element is connected to the housing by means of one or multiple locking elements, which are in positive locking engagement with one or multiple first counter-locking elements formed on the housing. Due to the positive locking engagement the fixing element is held on the housing. The fixing element herein assumes a position on the housing which allows for an easy placement of the catheter on the connector of the port.

For fixing the catheter on the port after placing the catheter on the connector, the fixing element can be transferred from its preassembled state into the assembled state. In the assembled state the one or multiple locking elements of the fixing element are in positive locking engagement with one or multiple second counter-locking elements formed on the housing, such that in the assembled state the fixing element is fixedly connected to the housing. Herein, in the assembled state the fixing element acts, by means of its clamping element, onto the catheter such that the catheter is fixed with respect to the shaft portion of the connector and hence with respect to the port.

For transferring the fixing element from the preassembled state to the assembled state, a user simply needs to move the fixing element in the fixing direction with respect to the housing. Because this may be achieved by a simple pushing action, involving in particular no additional step of placing the fixing element on the housing, a user can perform the fixing of the catheter on the port by means of a one-hand action, hence making the handling easy and simple for a user and avoiding the risk of losing the fixing element during use.

In one embodiment, the housing comprises an opening, wherein the shaft portion is placed at least partially in said opening of the housing. The opening, in the shape of a bore, extends in the housing along the fixing direction, the opening having for example a generally cylindrical shape and the shaft portion extending concentrically to the axis of the opening.

The clamping element of the fixing element herein, in one embodiment, is movable in said opening of the housing by moving the fixing element in between its preassembled state and assembled state. By moving the clamping element in the opening the clamping element is moved with respect to the shaft portion in order to act onto the catheter placed on the shaft portion. The clamping element for example may have a cylindrical shape and may be guided within the opening of the housing such that the clamping element is movable within the opening of the housing along a defined movement path.

In one embodiment the clamping element comprises a clamping opening extending along the fixing direction and receiving the shaft portion of the connector. The shaft portion hence, at least in the assembled state of the fixing element, reaches into the clamping opening of the clamping element, the clamping element circumferentially extending about the shaft portion and hence circumferentially enclosing the shaft portion. By moving the fixing element in between the preassembled state and the assembled state the clamping element is moved with respect to the shaft portion axially along the fixing direction, the clamping opening of the clamping element hence being displaced with respect to the shaft portion.

The clamping element beneficially is rigid and not elastically deformed when moving the fixing element from the preassembled state to the assembled state.

In one embodiment, the clamping element comprises a shoulder portion formed within the clamping opening. The shoulder portion in particular is formed by a portion of the clamping element protruding into the clamping opening, such that the shoulder portion comes into engagement with the catheter when the fixing element is in its assembled state. The shoulder portion in particular may be formed in between a first, widened portion of the clamping opening and a second, narrowed portion of the clamping opening, the first, widened portion and the second, narrowed portion being displaced with respect to each other along the fixing direction and the shoulder portion being formed axially in between the first, widened portion and the second, narrowed portion. The shoulder portion hence is formed by a transition region in between the first, widened portion and the second, narrowed portion of the clamping opening, the shoulder portion having a shape for interacting with the catheter placed on the connector when the fixing element is in the assembled state.

To allow a clamping of the catheter on the connector, the shaft portion, in one embodiment, comprises a widened section and a tip section axially adjoining the widened section. The widened section of the shaft portion herein is placed within the first, widened portion of the clamping opening and the tip section reaches into the second, narrowed portion of the clamping opening. By moving the fixing element from the preassembled state to the assembled state the shoulder portion formed within the clamping opening is approached towards a shoulder region of the shaft portion formed in between the widened section and the tip section, such that the catheter placed on the connector is clamped in between the shoulder portion of the clamping element and the shoulder region of the shaft portion, hence providing for a clamping connection and fixation of the catheter on the shaft portion of the connector. In this way the catheter is fixed on the connector when the fixing element is in the assembled state, such that the catheter cannot be removed from the port, at least not without reverting the fixing element from the assembled state to the preassembled state.

The at least one locking element of the fixing element beneficially is elastically deformable such that the at least one locking element may elastically snap into engagement with the second counter-locking element when reaching the assembled state. The positive locking engagement hence is established in an automatic fashion when reaching the assembled state.

The connection of the catheter to the port herein may be releasable. For this, the fixing element can be released from its assembled state and can be moved back into the preassembled state, allowing to disconnect the catheter from the port.

The housing comprises at least one locking cavity in which the first and second counter-locking elements are formed for interacting with the at least one locking element of the fixing element. The at least one locking element herein, in one embodiment, protrudes from the body portion of the fixing element along the fixing direction and reaches into the at least one locking cavity formed on the housing in order to engage with the first counter-locking element in the preassembled state of the fixing element and with the second counter-locking element in the assembled state of the locking cavity. The at least one locking cavity is, in one embodiment, spatially separate from the opening of the housing receiving the shaft portion of the connector. In particular, in one embodiment two locking cavities may be formed on the housing, one at each side of the opening of the housing receiving the shaft portion of the connector.

In the assembled state of the fixing element the fixing element is fixedly connected to the housing of the port by means of the at least one locking element engaging with the second counter-locking element of the housing. In order to allow for a releasing of the fixing element from the assembled state, in one embodiment the second counter-locking element formed on the housing comprises a slanted face extending at a slanted angle with respect to the fixing direction, the at least one locking element in the assembled state of the fixing element being in engagement with the slanted face by abutting the slanted face in a direction opposite to the fixing direction. Because the slanted face extends at an angle with respect to the fixing direction the fixing element may be pulled out of its assembled state by applying a suitable force to the fixing element. The slanted face herein is slanted such that the at least one locking element of the fixing element may run up the slanted face when applying a force opposite to the fixing direction to the fixing element such that the connection in between the fixing element and the housing may be released by applying a suitable force to the fixing element.

In order to provide for an additional fixation of the fixing element to the housing in the assembled state, additional snap elements may be formed on the fixing element. For example, the body portion may comprise an end section at a far end with respect to the clamping element, for example at a leg protruding from the clamping element in a direction transverse to the fixing direction, the end section comprising a snap element which, in the assembled state of the fixing element, engages with a counter-snap element formed on the housing. By means of the additional snap element hence an additional positive locking connection in between the fixing element and the housing is established in order to secure the fixing element on the housing in the assembled state.

The end section herein may be elastically deformable with respect to other portions of the body portion such that by deforming the end section the engagement of the snap element with the counter-snap element of the housing may be released in order to revert the fixing element from its assembled state to the preassembled state.

In one embodiment, at least one of the housing and the fixing element comprises a radiopaque marker having an X-ray discernible marking structure. The radiopaque marker may for example have the shape of an inlay element manufactured from a radiopaque material, for example stainless steel. Such inlay element may for example be injection molded with the housing or the fixing element, wherein it is conceivable that both the housing and the fixing element each comprise an inlay element of this kind.

Instead of providing an inlay element injection molded with the housing and/or the fixing element, a radiopaque marker may also be formed by providing a coating or (embedded) layer, for example by printing, of a radiopaque material on or within the housing and/or the fixing element to form a marking structure on the housing and/or the fixing element.

The marking structure may for example comprise alphabetical letters or numbers to provide for a marking which for example is visible in an X-ray or CT image. Such letters or numbers may be formed as positives (i.e., the radiopaque material of the marking structure positively forming the letters and numbers) or negatives (i.e., the radiopaque material having cutouts forming the letters or numbers).

When both the fixing element and the housing each comprise a radiopaque marker, it is also conceivable to identify in an X-ray or CT image, by checking the relative placement of the radiopaque markers of the fixing element and the housing, whether the fixing element is correctly placed on the housing and in particular is in the assembled state, such that it can be verified whether the catheter is correctly connected to the port even with the port being implanted into a patient.

In one embodiment, the port comprises an indicator comprising a first indicator portion placed on the housing and a second indicator portion placed on the fixing element. The first indicator portion assumes a first position relative to the second indicator portion in the preassembled state of the fixing element, and assumes a second position relative to the second indicator portion in the assembled state of the fixing element. The indicator portions herein are beneficially visible on the outside of the port and hence serve to visually indicate to a user whether the fixing element has correctly been transferred to the assembled state.

The first indicator portion and the second indicator portion may each have the shape of a strip or line on the housing respectively the fixing element. In the assembled state of the fixing element the first indicator portion and the second indicator portion may for example be brought into alignment with one another, such that a user immediately - from visual inspection - is informed whether the fixing element has correctly been transferred to its assembled state.

The first indicator portion and the second indicator portion may be formed by a suitable marking on the housing respectively the fixing element visible to a user by visual inspection of the port from the outside. Alternatively or in addition the first indicator portion and the second indicator portion may each be formed by a suitable radiopaque material, such that the first indicator portion and the second indicator portion may be visible in an X-ray or CT image.

An indicator comprising two indicator portions may be placed on one side of the port. It also is conceivable however to place one indicator on each side of the port, each indicator comprising a first indicator portion placed on the housing and a second indicator portion placed on the fixing element.

Beneficially, the closure element, for example constituted by a needle-penetrable membrane or a septum, has an area which is relatively large with respect to the dimensions of the port, in particular with respect to the height of the port. In one embodiment, the relation of the diameter of the area of the closure element accessible from the outside and the height of the port may lie in a range between 1:1 and 1 :0.8, the diameter of the puncturing area hence being as large as or even larger than the height of the port. This allows a user to easily access the cavity formed in the housing of the port by puncturing the closure element, reducing the risk for missing the puncturing area of the closure element.

In one embodiment, the port has an outer shape defined by the housing and the closure element attached to the housing, wherein at least one adapter piece in a pre-use state is separate from the housing and is attachable to the housing to alter the outer shape of the port, wherein the port is operational with or without the at least one adapter piece attached to the housing. Hence, the port as such is operational and hence can be used and implanted into a patient for medical treatment. The housing of the port herein defines an outer shape of the port, which however may be altered by attaching one or multiple adapter pieces to the housing. By using one or multiple adapter pieces being attached to the housing the size of the port hence can be varied. The port can be used without any adapter pieces and as such has a rather small size. By attaching one or multiple adapter pieces to the housing of the port the size of the port can be increased and the port hence can be adapted for a specific use in a particular patient.

By providing one or multiple adapter pieces attachable to the housing a port system is provided which in a variable way can be used in different patients. A port having a rather small size may be provided by not attaching any adapter pieces to the housing. Such small port may be used in particular in small patients. To increase the size of the port one or multiple adapter pieces may be attached to the housing such that the outer shape of the port is altered and adapted in particular for use in bigger patients.

The outer shape of the port in particular is defined by a maximum height, a maximum length and a maximum width, wherein the maximum height is measured along a direction perpendicular to a plane of the extension of a lower face of the housing opposite the access opening, and the maximum length and the maximum width are measured in directions parallel to the plane of extension of the base plate. If no adapter piece is attached to the housing, the maximum length and the maximum width of the port herein may be defined by the extension of the lower face.

In an advantageous embodiment the at least one adapter piece is attached to the lower face. In a mounted state the adapter piece preferably extends parallel to the lower face and is connected immediately to the lower face. The adapter piece hence adds to the height of the port and furthermore may have a larger length and/or width than the lower face, such that also the maximum length and/or maximum width of the port are increased.

In addition, it is conceivable that other adapter pieces can be attached to other faces of the housing such that the shape and size of the port may be adapted at other faces of the housing.

The port may have an insertion piece inserted into the housing and forming the cavity. The insertion piece may function as an inlay providing the cavity, wherein the insertion piece preferably is made of a material resistant against the medical fluid to be used in the port, for example a metallic material or a ceramic material. The connector herein is in fluid connection with the cavity formed by the insertion piece.

The needle-penetrable closure element in the shape of a membrane or septum serves as an access means for introducing a medical fluid into the cavity of the port. For this, a puncture needle may be used for puncturing the closure element such that a medical fluid through the closure element can be filled into the cavity of the port.

A port of the kind described herein may be used in all applications and medical fields in which there exists a need for providing an intravenous access, for the purpose of infusion, injection, transfusion, power injection of X-ray media, or venous blood collection. In particular, a port of the kind described herein may be usable for injections related to cancer diseases, in particular for chemotherapies, in conjunction with parenteral nutrition and/or pain therapy.

The idea underlying the invention shall subsequently be described in more detail with respect to the embodiments shown in the figures. Herein:
- Fig. 1: shows a view of an embodiment of a port for a catheter for subcutaneous implantation into a patient;
- Fig. 2: shows a sectional view, along a longitudinal-vertical plane, of the port;
- Fig. 3A: shows a sectional view, along a horizontal plane, of the port, in a preassembled state of a fixing element for fixing a catheter on a housing of the port;
- Fig. 3B: shows the sectional view of Fig. 3A, in an assembled state of the fixing element;
- Fig. 4: shows a sectional view of the port in the assembled state of the fixing element;
- Fig. 5: shows yet another sectional view of the port in the preassembled state of the fixing element;
- Fig. 6: shows a sectional view of an embodiment of a port comprising a radiopaque marker;
- Fig. 7A: shows a schematic view of a port, with no adapter pieces attached to the housing of the port;
- Fig. 7B: shows the port, with one adapter piece attached to a lower face of the housing of the port; and
- Fig. 7C: shows the port with two adapter pieces attached.

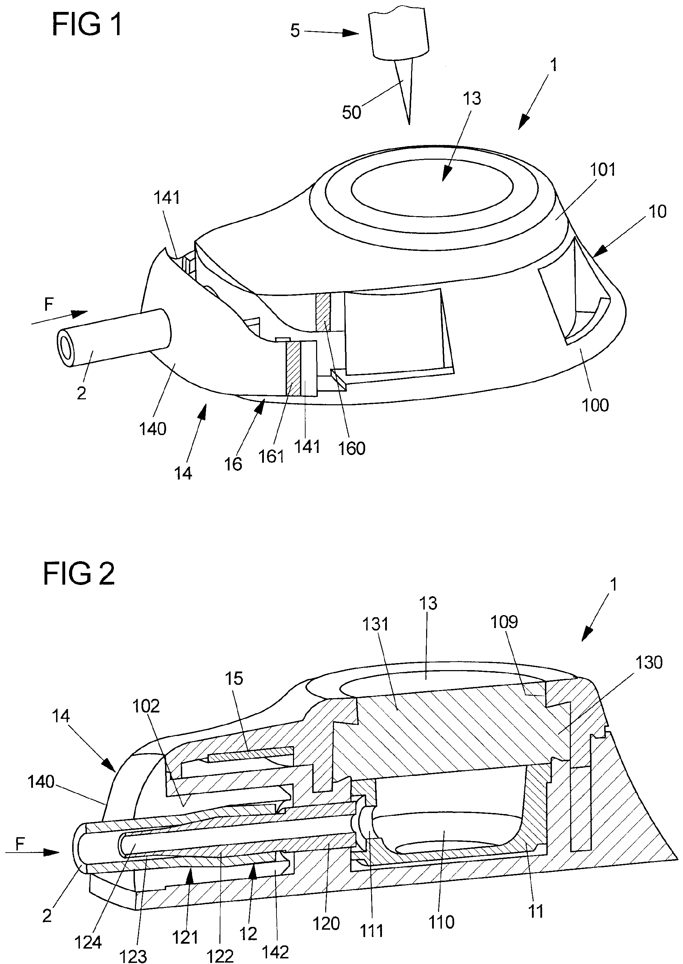

Fig. 1 and 2 show views of a port 1 having a housing 10 and a needle-penetrable closure element 13 attached to the housing 10. A catheter 2 is arranged on the housing 10 and extends from the port 1, the catheter 2 being fixable to the housing 10 by means of a fixing element 14.

The port 1 may be implanted subcutaneously under a skin of a patient. Herein, the port 1 is constituted to hold a medical fluid which via the catheter 2 attached to the housing 10 of the port 1 is to be administered to the patient, for example by guiding it into the venous or arterial system of the patient.

The housing 10 comprises a first housing part 100 and a second housing part 101 attached to each other. In-between the housing parts 100, 101 an insertion piece 11 is arranged which defines a cavity 110 for providing a reservoir to hold a medical fluid and which preferably is made of a material resistant to a medical fluid to be received within the cavity 110, for example a metallic or ceramic material.

The second housing part 101, at its top face, defines an access opening 109 in which the closure element 13 in the shape of a membrane or septum element is placed. The closure element 13, via an edge 130, is clampingly fixed in between the housing parts 100, 101 of the housing 10 and seals the access opening 109 towards the outside. The closure element 13, at a central portion 131, may be punctured by a puncture needle 50 of a delivery device 5, for example a syringe, such that via the puncture needle 50 a medical fluid may be inserted into the cavity 110. Through the closure element 13, hence, in an implanted state of the port 1, the cavity 110 may be refilled by introducing a medical fluid into the cavity 110, such that the port 1 may be used over a long period of time within a patient.

The port 1 comprises a connector 12 which, by means of a fixation portion 120 is fixed to the first housing part 100 and reaches into an opening 102 formed as a bore within the first housing part 100 of the housing 10. The connector 12 has a needlelike shape and forms a shaft portion 121, which is placed within the opening 102 of the housing 10 and is configured to receive the catheter 2. A flow channel 124 extends longitudinally through the connector 12 and is in fluid connection, via a flow opening 111 of the insert piece 11, with the cavity 110 formed within the insert piece 11.

For connecting a catheter 2 to the port 1, the catheter 2 is placed on the connector 12 such that the shaft portion 121 is inserted into the catheter 2, as visible for example from Fig. 2. Hence, the catheter 2 comes into fluid connection with the cavity 110, such that fluid from the cavity 110 may access the catheter 2 or vice versa.

The fixing element 14 comprises a body portion 140 in the shape of a front section, a clamping element 142 protruding along a fixing direction F from the body portion 140. The clamping element 142 has a cylindrical shape and is placed within the opening 102 formed within the housing 10 such that, by moving the fixing element 14 with respect to the housing 10, the clamping element 142 can be moved within the opening 102 with respect to the shaft portion 121 extending within the opening 102.

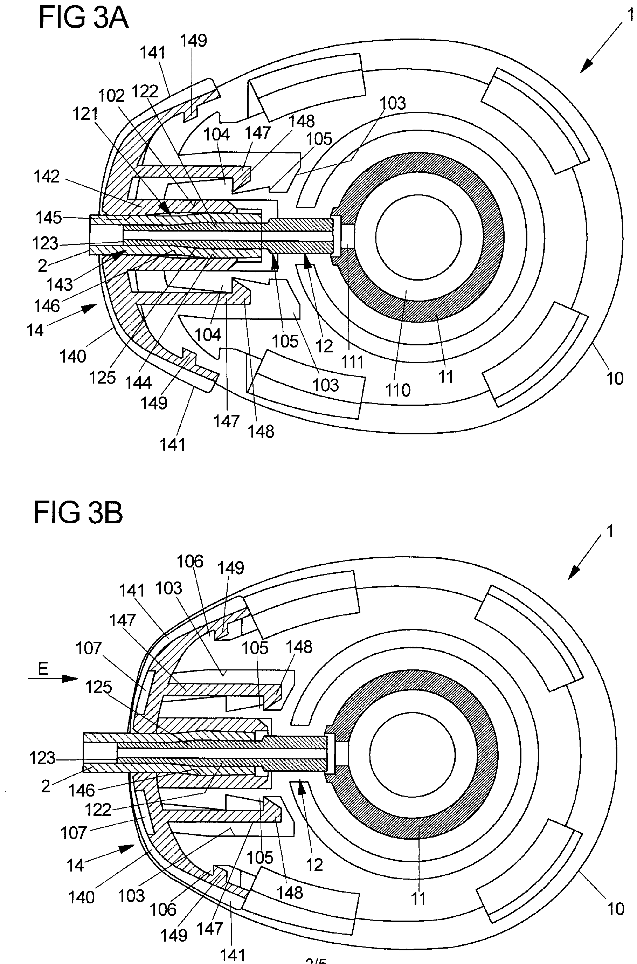

The clamping element 142 comprises a clamping opening 143 into which the shaft portion 121 of the connector 12 reaches, as visible for example from Figs. 3A, 3B and Figs. 4 and 5. The clamping opening 143 herein has a widened portion 144 and a narrowed portion 145, a shoulder portion 146 being formed in a transition region axially in between the widened portion 144 and the narrowed portion 145.

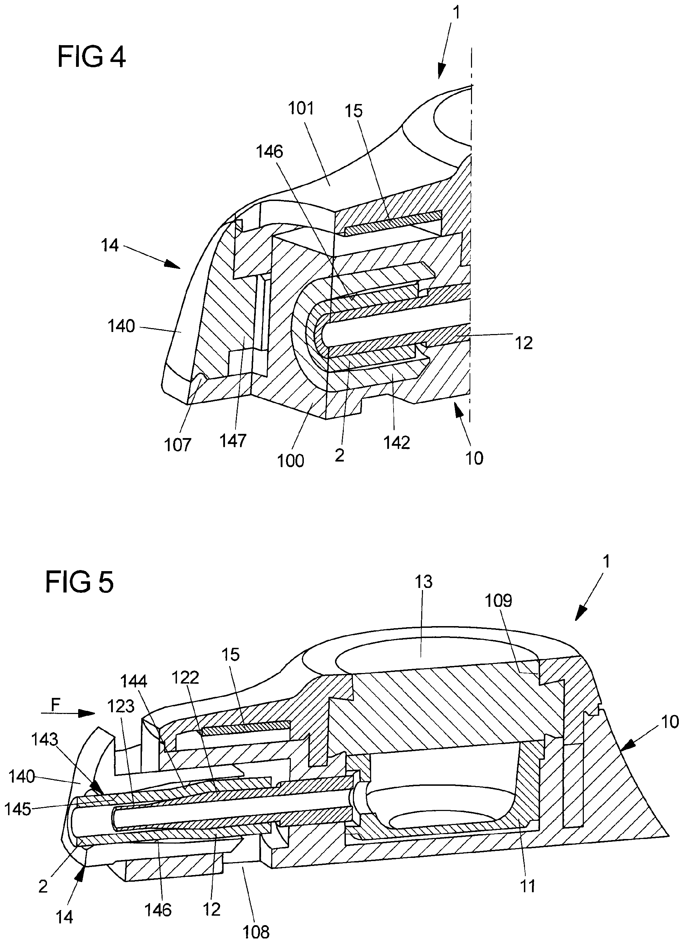

The diameter of the narrowed portion 145 of the clamping opening 143 roughly corresponds to the outer diameter of the catheter 2, as visible for example from Fig. 5. The diameter of the widened portion 144 in comparison is larger. The shaft portion 121 of the connector 12 forms a widened section 122 extending in the widened portion 144 of the clamping opening 143. A tip section 123 adjoins the widened section 122, a shoulder region 125 being formed in between the tip section 123 and the widened section 122. The tip section 123 reaches into the narrowed portion 145 of the clamping opening 143, as visible from Fig. 5.

The fixing element 14 comprises two locking elements 147 formed on and protruding from the body portion 140, as visible for example from Figs. 3A, 3B. The locking elements 147 in the shape of locking fingers reach into locking cavities 103 formed within the first housing part 100 of the housing 10, one locking element 147 being placed at each side of the clamping element 142, as visible from Fig. 3A and 3B.

Each locking element 147 comprises, at a distal end with respect to the body portion 140, a locking nose 148 which serves to establish a positive locking connection between the fixing element 14 in a preassembled state (Fig. 3A) and in an assembled state (Fig. 3B). In the preassembled state (Fig. 3A) the locking noses 148 of the locking elements 147 each are in engagement with a first counter-locking element 104 formed within the corresponding locking cavity 103. By moving the fixing element 14 in the fixing direction F with respect to the housing 10 the fixing element 14 is transferred from the preassembled state into the assembled state (Fig. 3B), in which the locking noses 148 of the locking elements 147 each are in positive locking engagement with a second counter-locking element 105 formed within the corresponding locking cavity 103, such that also in the assembled state a positive locking engagement and fixation of the fixing element 14 on the housing 10 is established.

Due to the positive locking engagement of the locking elements 147 with the housing 10 the fixing element 14 already in the preassembled state is held on the housing 10, such that the fixing element 14 cannot be lost. By transferring the fixing element 14 into the assembled state the catheter 2 is fixed on the connector 12 in that the shoulder portion 146 formed within the clamping opening 143 is approached the shoulder region 125 formed in between the tip section 123 and the widened section 122 on the shaft portion 121 such that the catheter 2 is clamped in between the shoulder portion 146 and the shoulder region 125, as visible from Fig. 3B. The catheter 2 hence is clamped on the shaft portion 121 and hence is fixed on the connector 12.

Because the fixing element 14 can be transferred from its preassembled state into the assembled state by means of a simple pushing action by moving the fixing element 14 in the fixing direction F with respect to the housing 10, a user can act onto the fixing element 14 using only one hand, such that the fixation of the catheter 2 on the port 1 becomes easy and intuitive.

The locking elements 147 in the shape of the locking fingers are elastically deformable. When transferring the fixing element 14 from the preassembled state (Fig. 3A) to the assembled state (Fig. 3B) the locking elements 147 hence elastically are deformed and snap into place with the second counter-locking elements 105, such that in the assembled state the fixing element 14 is fixed on the housing 10.

The fixing element 14, at end sections 141, comprises snap elements 149 which in the assembled state engage with corresponding counter-snap elements 106 formed on the first housing part 100 of the housing 10. In addition, locking elements 107 are formed on the first housing part 100 to engage with the body portion 140 of the fixing element 14 in the assembled state of the fixing element 14, as visible from Fig. 4 in view of Fig. 3B. In the assembled state the fixing element 14 hence additionally is fixed with respect to the housing 10 and hence is held in position with respect to the housing 10.

If the catheter 2 shall be released from the port 1, the fixing element 14 can be reverted from its assembled state to the preassembled state. For this, the end sections 141 can elastically be moved outwards such that the engagement between the snap elements 149 and the counter-snap elements 106 is released. Furthermore, the second counter-locking elements 105 each comprise a slanted face with which the locking nose 148 of the associated locking element 147 is in engagement in the assembled state, as visible in Fig. 3B. Because the counter-locking elements are slanted, the fixing element 14 can be reverted to the preassembled state by applying a suitable force on the fixing element 14, the force causing the locking noses 148 to run up the counter-locking elements 105 such that the positive locking engagement in between the locking elements 147 and the counter-locking elements 105 in the assembled state is released.

The fixing element 14 hence may be reverted to the preassembled state. When the fixing element 14 again is in the preassembled state, the catheter 2 no longer is clamped with respect to the connector 12 and hence can be pulled off the connector 12.

As visible for example from Fig. 5, the first housing part 100 of the housing 10 comprises, at its lower face opposite the closure element 13, an inspection opening 108 which allows to visually inspect the port 1 to verify whether the fixing element 14 correctly assumes the assembled state and correctly fixes the catheter 2 on the connector 12.

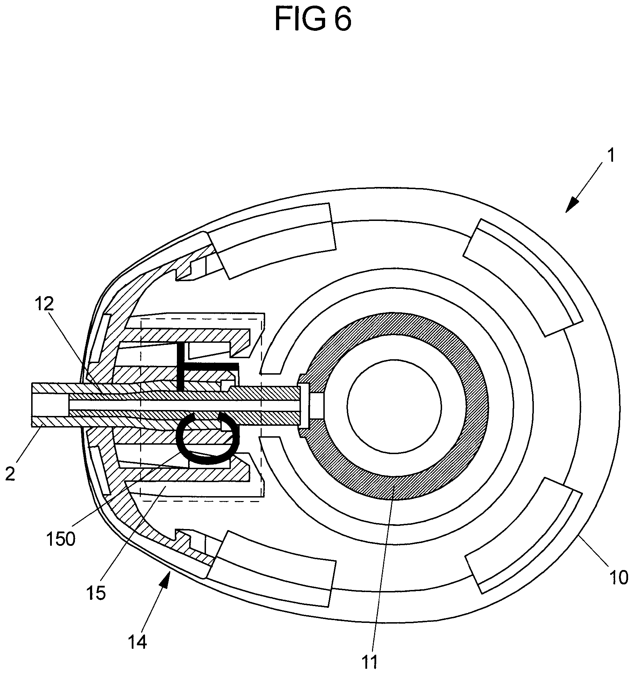

The port 1, in one embodiment, comprises a marker 15 comprising an X-ray discernible marking structure 150 as shown in Fig. 6. In the embodiment of Figs. 1 to 5 the marker 15 is formed by an inlay element placed on the housing 10, for example injection molded with one of the housing parts 100, 101. The inlay element is formed from a radiopaque material, such as stainless steel, and forms the marking structure 150 which is visible for example on an X-ray or CT image.

Alternatively or additionally, a marker 15 may be placed on the fixing element 14. A marking structure 150 hence can also be provided on the fixing element 14.

The marker 15 may be formed by an inlay element. It however also is possible to form the marker 15 by a suitable radiopaque coating formed on a face of one of the housing parts 100, 101 or the fixing element 14 or by a radiopaque layer embedded within one of the housing parts 100, 101 or the fixing element 14.

The marking structure 150 may comprise alphabetical letters or numbers, as visible in Fig. 6, or may comprise any other structure providing information which are visible for example in an X-ray or CT image.

As visible from Fig. 1, the port 1 comprises an indicator 16 formed by two indicator portions 160, 161, one indicator portion 160 being placed on the outside of the housing 10, and the other indicator portion 161 being placed on the outside of the fixing element 14. The indicator portions 160, 161 serve to indicate to a user whether the fixing element 14 is in its preassembled state (as shown in Fig. 1) or has been correctly transferred to the assembled state. In the assembled state, herein, the indicator portions 160, 161 beneficially are aligned with one another such that a user by visual inspection can immediately see whether the fixing element 14 has correctly reached its assembled state.

The indicator portions 160, 161 may each be formed by a suitable color marking visible to a user by visual inspection. In addition or alternatively, the indicator portions 160, 161 may be formed from a radiopaque material, such that the indicator portions 160, 161 and their relative position to another may also be visible in an X-ray or CT image.

An indicator 16 comprising two indicator portions 160, 161 may be placed on one side of the port 1 only, as shown in Fig. 1. An indicator 16 of this kind may however also be placed on each side of the port 1, such that the port 1 comprises indicators 16 on opposite sides.

As visible from for example Fig. 1, the closure element 13 in the shape of a membrane element or septum comprises a rather large diameter, in comparison to the overall dimensions of the port 1. The closure element 13 hence provides for a relatively large access area allowing a puncturing by a puncture needle 50. For example, the ratio between the diameter of the access area and the height of the port 1 may be in the range of 1:1 to 1:0.8. In this way an easy access of the port 1 by means of a puncture needle 50 is possible, reducing the risk for missing the access area and hence reducing the risk for infections or extravasation.

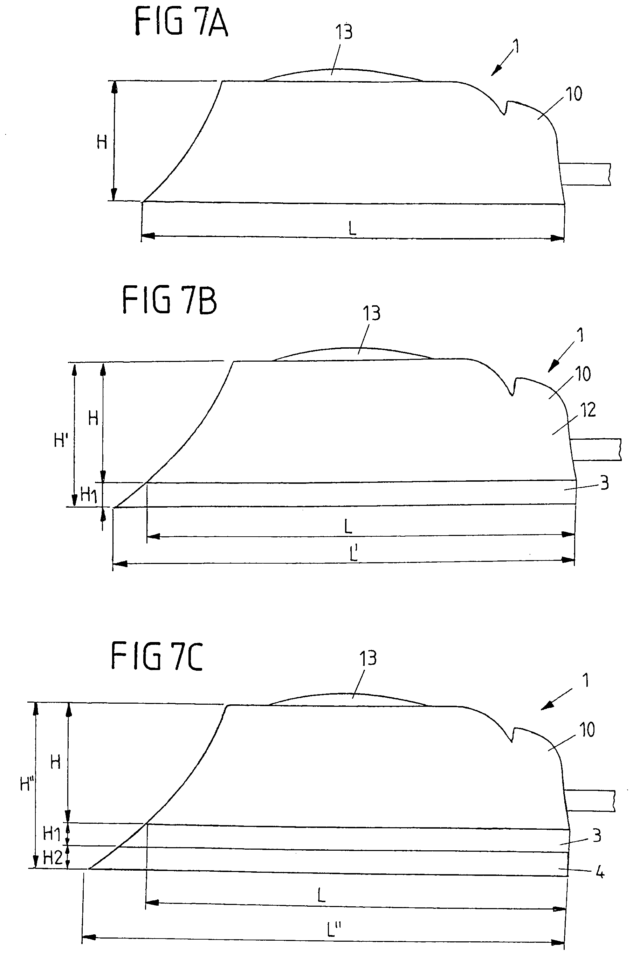

As shown in Figs. 7A to 7C, the port 1 may be used together with one or multiple adapter pieces 3, 4 attached to a lower face of the housing 10 opposite the closure element 13. The adapter pieces 3, 4, for the operation of the port 1, have no immediate function, but serve to alter the outer shape of the port 1 in order to adapt the port 1 to specific requirements for implantation into a particular patient.

Namely, the port 1, without the adapter piece 3 attached, has a maximum width and a maximum length L defined by the extension of the lower face of the housing 10 (see Fig. 7A). In addition, the port 1 has a height H defined by the height of the housing 10. By attaching one or multiple adapter pieces 3, 4 to the lower face of the housing 10, the width as well as the length L', L" and the height H', H" of the port 1 are increased such that the port 1 has a larger overall size and in this way is adapted for use for example in a bigger patient.

As shown in the schematic drawings of Fig. 7A to 7C, the port 1 may be used in connection with no adapter piece (Fig. 7A), with one adapter piece 3 (Fig. 7B) or with two adapter pieces 3, 4 (Fig. 7C), wherein it is also conceivable to use even more than two adapter pieces 3, 4 or other adapter pieces than the ones shown. As shown in Fig. 7A to 7C, by attaching one or multiple adapter pieces 3, 4 to the housing 10 of the port 1, the outer shape of the port 1 may be altered to adapt the port 1 for use in different patients. Namely, the housing 10 as such may define an overall height H and overall length L of the port 1, as shown in Fig. 7A. By attaching one adapter piece 3 to the base plate 12 of the housing 10, the overall height H' and the overall length L' of the port 1 may be increased. By attaching a second adapter piece 4 to the first adapter piece 3 as shown in Fig. 7C, the overall height H" and the overall length L" of the port 1 may further be increased, such that the port 1 in a variable fashion may be adapted for use in different patients.

The port is not limited to the dimension and shape as shown in the figures. A port of the type described herein may be used in the context of different applications, for example to allow for an infusion, injection, transfusion, power injection of X-ray media, and/or venous blood collection using an implanted port. A port of the kind described herein may in particular be usable for the treatment of cancer diseases, for example in the context of chemotherapy, possibly in conjunction with parenteral nutrition and/or pain therapy.

### List of reference numerals

- 1: Port
- 10: Housing
- 100, 101: Housing part
- 102: Opening
- 103: Locking cavity
- 104, 105: Counter-locking element
- 106: Counter-snap element
- 107: Locking element
- 108: Inspection opening
- 109: Access opening
- 11: Insertion piece
- 110: Cavity
- 111: Flow opening
- 12: Connector
- 120: Fixation portion
- 121: Shaft portion
- 122: Widened section
- 123: Tip section
- 124: Flow channel
- 125: Shoulder region (transition region)
- 13: Closure element
- 130: Edge
- 131: Central portion
- 14: Fixing element
- 140: Body portion
- 141: End section
- 142: Clamping element
- 143: Clamping opening
- 144: Widened portion
- 145: Narrowed portion
- 146: Shoulder portion (transition region)
- 147: Locking element
- 148: Locking nose
- 149: Snap element
- 15: Marker
- 150: Marking structure
- 16: Indicator
- 160, 161: Indicator portion
- 2: Catheter
- 3: Adapter piece
- 4: Adapter piece
- 5: Delivery device
- 50: Puncture needle
- F: Fixing direction
- H, H', H", H1, H2: Height
- L, L', L": Length

## Claims

1. Port (1) for a catheter (2) for subcutaneous implantation into a patient, the port (1) comprising:
a housing (10), comprising at least one locking cavity (103),
a cavity (110) arranged in the housing (10) for receiving a fluid,
a needle-penetrable closure element (13) placed on the housing (10) and penetrable by a puncturing needle (50) for accessing the cavity (110),
a connector (12) for connecting the catheter (2) to the port (1), the connector (12) having a shaft portion (121) for receiving the catheter (2) and a flow channel (124) formed in the shaft portion (121) and being in fluid connection with the cavity (110), and
a fixing element (14) for fixing the catheter (2) on the connector (12), wherein
the fixing element (14) comprises a body portion (140), a clamping element (142) protruding from the body portion (140) along a fixing direction (F) and at least one locking element (147) formed on the body portion (140),
wherein the at least one locking element (147) protrudes from the body portion (140) along the fixing direction (F) and reaches into the at least one locking cavity (103),
wherein in a preassembled state of the fixing element (14), the fixing element (14) is preassembled on the housing (10) of the port (1) prior to placing the catheter (2) on the corresponding connector (12) of the port, wherein the at least one locking element (147) is in positive-locking engagement with a first counter-locking element (104) formed on the housing (10) to hold the fixing element (14) on the housing (10) in the pre-assembled state,
wherein in an assembled state of the fixing element (14) the at least one locking element (147) is in positive-locking engagement with a second counter-locking element (105) formed on the housing (10) to fix the fixing element (14) with respect to the housing (10) in the assembled state, and
wherein the fixing element (14) is movable with respect to the housing (10) along the fixing direction (F) for transferring the fixing element (14) from the pre-assembled state into the assembled state, the clamping element (142) comprises a clamping opening (143) extending along the fixing direction (F), wherein the shaft portion (121) at least in the assembled state of the fixing element (14) reaches into the clamping opening (143), and the clamping element (142) comprises a shoulder portion (146) formed within the clamping opening (143), wherein in the assembled state of the fixing element (14) the clamping element (142) is configured to act onto the catheter (2) by means of the shoulder portion (146) to fix the catheter (2) on the shaft portion (121) of the connector (12).

2. Port (1) according to claim 1, **characterized in that** the housing (10) comprises an opening (102), wherein the shaft portion (121) is placed at least partially in said opening (102) of the housing (10).

3. Port (1) according to claim 1 or 2, **characterized in that** the clamping element (142) is movable in said opening (102) of the housing (10) by moving the fixing element (14) from the pre-assembled state into the assembled state.

4. Port (1) according to claim 3, **characterized in that** the clamping opening (143) comprises a first, widened portion (144) and a second, narrowed portion (145), wherein the shoulder portion (146) is formed axially in between the first, widened portion (144) and the second, narrowed portion (145).

5. Port (1) according to claim 4, **characterized in that** the shaft portion (121) comprises a widened section (122) placed within the first, widened portion (144) of the clamping opening (143) and a tip section (123) reaching into the second, narrowed portion (145) of the clamping opening (143).

6. Port (1) according to claim 5, **characterized in that** the shaft portion (121) comprises a shoulder region (125) formed in between the widened section (122) and the tip section (123), wherein in the assembled state of the fixing element (14) the catheter (2) is clamped in between the shoulder portion (146) of the clamping element (142) and the shoulder region (125) of the shaft portion (121).

7. Port (1) according to at least one of the preceding claims, **characterized in that** the second counter-locking element (105) comprises a slanted face extending at a slanted angle with respect to the fixing direction (F), the at least one locking element (147) abutting the slanted face in a direction pointing opposite the fixing direction (F) in the assembled state of the fixing element (14).

8. Port (1) according to at least one of the preceding claims, **characterized in that** the body portion (140) comprises an end section (141) at a far end with respect to the clamping element (142), wherein the end section (141) comprises a snap element (149) which, in the assembled state of the fixing element (14), is in engagement with a counter-snap element (107) formed on the housing (10).

9. Port (1) according to at least one of the preceding claims, **characterized in that** at least one of the housing (10) and the fixing element (14) comprises a radiopaque marker (15) having an X-ray discernible marking structure (150).

10. Port (1) according to at least one of the preceding claims, **characterized by** an indicator (16) comprising a first indicator portion (160) placed on the housing (10) and a second indicator portion (161) placed on the fixing element (14), the first indicator portion (160) and the second indicator portion (161) assuming a first position relative to one another in the preassembled state of the fixing element (14) and a second position relative to one another in the assembled state of the fixing element (14), the second position visually indicating a correct attachment of the fixing element (14) to the housing (10) in the assembled state.

11. Port (1) according to one of the preceding claims, **characterized in that** the port (1) has an outer shape defined by the housing (10) and the closure element (13) attached to the housing (10), wherein at least one adapter piece (3, 4) is attachable to the housing (10) to alter the outer shape of the port (1), wherein the port (1) is operational with or without the at least one adapter piece (3, 4) attached to the housing (10).

12. Port (1) according to claim 11, **characterized in that** the outer shape is defined by a maximum height (H), a maximum length (L) and a maximum width, wherein the maximum height (H) is measured along a direction perpendicular to a plane of extension of a lower face of the housing (10) opposite the access opening (109) and the maximum length (L) and the maximum width are measured in directions parallel to the plane of extension of the lower face.

## Patentansprüche

1. Port (1) für einen Katheter (2) zur subkutanen Implantation in einen Patienten, wobei der Port (1) Folgendes umfasst:
ein Gehäuse (10), umfassend mindestens einen Verriegelungshohlraum (103),
einen im Gehäuse (10) angeordneten Hohlraum (110) zur Aufnahme eines Fluids,
ein nadeldurchdringbares Verschlusselement (13), das auf dem Gehäuse (10) platziert ist und von einer Punktionsnadel (50) durchdrungen werden kann, um auf den Hohlraum (110) zuzugreifen,
einen Anschluss (12) zum Verbinden des Katheters (2) mit dem Port (1), wobei der Anschluss (12) einen Schaftabschnitt (121) zur Aufnahme des Katheters (2) und einen im Schaftabschnitt (121) gebildeten Strömungskanal (124) aufweist und in Fluidverbindung mit dem Hohlraum (110) steht, und
ein Fixierelement (14) zum Fixieren des Katheters (2) am Anschluss (12), wobei
das Fixierelement (14) einen Körperabschnitt (140), ein entlang einer Fixierrichtung (F) vom Körperabschnitt (140) abstehendes Klemmelement (142) und mindestens ein am Körperabschnitt (140) gebildetes Verriegelungselement (147) umfasst,
wobei das mindestens eine Verriegelungselement (147) entlang der Fixierrichtung (F) vom Körperabschnitt (140) absteht und in den mindestens einen Verriegelungshohlraum (103) hineinreicht,
wobei in einem vormontierten Zustand des Fixierelements (14) das Fixierelement (14) vor dem Aufsetzen des Katheters (2) auf den entsprechenden Anschluss (12) des Ports am Gehäuse (10) des Ports (1) vormontiert ist, wobei das mindestens eine Verriegelungselement (147) mit einem am Gehäuse (10) gebildeten ersten Gegenverriegelungselement (104) in formschlüssigem Eingriff steht, um das Fixierelement (14) am Gehäuse (10) im vormontierten Zustand festzuhalten,
wobei in einem montierten Zustand des Fixierelements (14), das mindestens eine Verriegelungselement (147) mit einem am Gehäuse (10) gebildeten zweiten Gegenverriegelungselement (105) in formschlüssigem Eingriff steht, um das Fixierelement (14) gegenüber dem Gehäuse (10) im montierten Zustand zu fixieren, und
wobei das Fixierelement (14) bezüglich des Gehäuses (10) entlang der Fixierrichtung (F) beweglich ist, um das Fixierelement (14) vom vormontierten Zustand in den montierten Zustand zu überführen, wobei das Klemmelement (142) eine sich entlang der Fixierrichtung (F) erstreckende Klemmöffnung (143) umfasst, wobei der Schaftabschnitt (121) mindestens im montierten Zustand des Fixierelements (14) in die Klemmöffnung (143) hineinreicht, und
wobei das Klemmelement (142) einen innerhalb der Klemmöffnung (143) gebildeten Schulterabschnitt (146) umfasst, wobei das Klemmelement (142) im montierten Zustand des Fixierelements (14) ausgebildet ist, um über den Schulterabschnitt (146) auf den Katheter (2) einzuwirken, um den Katheter (2) am Schaftabschnitt (121) des Anschlusses (12) zu fixieren.

2. Port (1) nach Anspruch 1, **dadurch gekennzeichnet, dass** das Gehäuse (10) eine Öffnung (102) umfasst, wobei der Schaftabschnitt (121) mindestens teilweise in der Öffnung (102) des Gehäuses (10) platziert ist.

3. Port (1) nach Anspruch 1 oder 2, **dadurch gekennzeichnet, dass** das Klemmelement (142) in der Öffnung (102) des Gehäuses (10) bewegbar ist, indem das Fixierelement (14) vom vormontierten Zustand in den montierten Zustand bewegt wird.

4. Port (1) nach Anspruch 3, **dadurch gekennzeichnet, dass** die Klemmöffnung (143) einen ersten, erweiterten Abschnitt (144) und einen zweiten, verengten Abschnitt (145) umfasst, wobei der Schulterabschnitt (146) axial zwischen dem ersten, erweiterten Abschnitt (144) und dem zweiten, verengten Abschnitt (145) gebildet ist.

5. Port (1) nach Anspruch 4, **dadurch gekennzeichnet, dass** der Schaftabschnitt (121) einen erweiterten Abschnitt (122), der innerhalb des ersten, erweiterten Abschnitts (144) der Klemmöffnung (143) platziert ist, und einen Spitzenabschnitt (123) umfasst, der in den zweiten, verengten Abschnitt (145) der Klemmöffnung (143) hineinreicht.

6. Port (1) nach Anspruch 5, **dadurch gekennzeichnet, dass** der Schaftabschnitt (121) einen zwischen dem erweiterten Abschnitt (122) und dem Spitzenabschnitt (123) gebildeten Schulterbereich (125) umfasst, wobei im montierten Zustand des Fixierelements (14) der Katheter (2) zwischen dem Schulterabschnitt (146) des Klemmelements (142) und dem Schulterbereich (125) des Schaftabschnitts (121) eingeklemmt ist.

7. Port (1) nach mindestens einem der vorhergehenden Ansprüche, **dadurch gekennzeichnet, dass** das zweite Gegenverriegelungselement (105) eine in einem schrägen Winkel zur Fixierrichtung (F) verlaufende Schrägfläche umfasst, wobei das mindestens eine Verriegelungselement (147) in einer der Fixierrichtung (F) entgegengesetzten Richtung im montierten Zustand des Fixierelements (14) an der Schrägfläche anliegt.

8. Port (1) nach mindestens einem der vorhergehenden Ansprüche, **dadurch gekennzeichnet, dass** der Körperabschnitt (140) an einem in Bezug auf das Klemmelement (142) entfernten Ende einen Endabschnitt (141) umfasst, wobei der Endabschnitt (141) ein Schnappelement (149) umfasst, das im montierten Zustand des Fixierelements (14) mit einem am Gehäuse (10) gebildeten Gegenschnappelement (107) im Eingriff steht.

9. Port (1) nach mindestens einem der vorhergehenden Ansprüche, **dadurch gekennzeichnet, dass** mindestens eines von dem Gehäuse (10) und dem Fixierelement (14) einen röntgendichten Marker (15) umfasst, das eine röntgensichtbare Markierungsstruktur (150) aufweist.

10. Port (1) nach mindestens einem der vorhergehenden Ansprüche, **gekennzeichnet durch** einen Indikator (16), der einen ersten Indikatorabschnitt (160) umfasst, der an dem Gehäuse (10) platziert ist, und einen zweiten Indikatorabschnitt (161), der an dem Fixierelement (14) platziert ist, wobei der erste Indikatorabschnitt (160) und der zweite Indikatorabschnitt ( 161) im vormontierten Zustand des Fixierelements (14) eine erste Position zueinander und im montierten Zustand des Fixierelements (14) eine zweite Position zueinander einnehmen, wobei die zweite Position eine korrekte Befestigung des Fixierelements (14) am Gehäuse (10) im montierten Zustand optisch anzeigt.

11. Port (1) nach einem der vorhergehenden Ansprüche, **dadurch gekennzeichnet, dass** der Port (1) eine durch das Gehäuse (10) und das am Gehäuse (10) befestigte Verschlusselement (13) definierte Außenform aufweist, wobei mindestens ein Adapterstück (3, 4) am Gehäuse (10) zur Veränderung der äußeren Form des Ports (1) befestigbar ist, wobei der Port (1) mit oder ohne das mindestens eine am Gehäuse (10) befestigte Adapterstück (3, 4) funktionsfähig ist.

12. Port (1) nach Anspruch 11, **dadurch gekennzeichnet, dass** die äußere Form durch eine maximale Höhe (H), eine maximale Länge (L) und eine maximale Breite definiert ist, wobei die maximale Höhe (H) entlang einer Richtung senkrecht zu einer Erstreckungsebene einer Unterseite des Gehäuses (10) gegenüber der Zugangsöffnung (109) gemessen wird und die maximale Länge (L) und die maximale Breite in Richtungen parallel zur Erstreckungsebene der Unterseite gemessen werden.

## Revendications

1. Orifice (1) pour un cathéter (2) pour implantation sous-cutanée chez un patient, l'orifice (1) comprenant :
un boîtier (10), comprenant au moins une cavité de verrouillage (103), une cavité (110) agencée dans le boîtier (10) pour recevoir un fluide,
un élément de fermeture pénétrable par une aiguille (13) placé sur le boîtier (10) et pénétrable par une aiguille perforante (50) pour accéder à la cavité (110),
un connecteur (12) pour connecter le cathéter (2) à l'orifice (1), le connecteur (12) ayant une partie de tige (121) pour recevoir le cathéter (2) et un canal d'écoulement (124) formé dans la partie de tige (121) et étant en connexion fluidique avec la cavité (110), et
un élément de fixation (14) pour fixer le cathéter (2) sur le connecteur (12), dans lequel
l'élément de fixation (14) comprend une partie corps (140), un élément de serrage (142) faisant saillie depuis la partie corps (140) le long d'une direction de fixation (F) et au moins un élément de verrouillage (147) formé sur la partie corps (140),
dans lequel le au moins un élément de verrouillage (147) fait saillie depuis la partie corps (140) le long de la direction de fixation (F) et atteint l'intérieur de la au moins une cavité de verrouillage (103),
dans lequel dans un état pré-assemblé de l'élément de fixation (14), l'élément de fixation (14) est pré-assemblé sur le boîtier (10) de l'orifice (1) avant de placer le cathéter (2) sur le connecteur (12) correspondant de l'orifice, dans lequel le au moins un élément de verrouillage (147) est en prise de verrouillage positif avec un premier élément de contre-verrouillage (104) formé sur le boîtier (10) pour maintenir l'élément de fixation (14) sur le boîtier (10) dans l'état pré-assemblé,
dans lequel, dans un état assemblé de l'élément de fixation (14), le au moins un élément de verrouillage (147) est en prise de verrouillage positif avec un second élément de contre-verrouillage (105) formé sur le boîtier (10) pour fixer l'élément de fixation (14) par rapport au boîtier (10) dans l'état assemblé, et dans lequel l'élément de fixation (14) est mobile par rapport au boîtier (10) le long de la direction de fixation (F) pour transférer l'élément de fixation (14) de l'état pré-assemblé à l'état assemblé, l'élément de serrage (142) comprend une ouverture de serrage (143) s'étendant le long de la direction de fixation (F), dans lequel la partie de tige (121), au moins dans l'état assemblé de l'élément de fixation (14), atteint l'intérieur de l'ouverture de serrage (143), et l'élément de serrage (142) comprend une partie d'épaulement (146) formée à l'intérieur de l'ouverture de serrage (143), dans lequel, dans l'état assemblé de l'élément de fixation (14), l'élément de serrage (142) est conçu pour agir sur le cathéter (2) au moyen de la partie d'épaulement (146) pour fixer le cathéter (2) sur la partie de tige (121) du connecteur (12).

2. Orifice (1) selon la revendication 1, **caractérisé en ce que** le boîtier (10) comprend une ouverture (102), dans lequel la partie de tige (121) est placée au moins partiellement dans ladite ouverture (102) du boîtier (10).

3. Orifice (1) selon la revendication 1 ou 2, **caractérisé en ce que** l'élément de serrage (142) est mobile dans ladite ouverture (102) du boîtier (10) en déplaçant l'élément de fixation (14) de l'état pré-assemblé à l'état assemblé.

4. Orifice (1) selon la revendication 3, **caractérisé en ce que** l'ouverture de serrage (143) comprend une première, partie élargie (144) et une seconde, partie rétrécie (145), dans lequel la partie d'épaulement (146) est formée axialement entre la première, partie élargie (144) et la seconde, partie rétrécie (145).

5. Orifice (1) selon la revendication 4, **caractérisé en ce que** la partie de tige (121) comprend une section élargie (122) placée à l'intérieur de la première, partie élargie (144) de l'ouverture de serrage (143) et une section de pointe (123) atteignant la seconde, partie rétrécie (145) de l'ouverture de serrage (143).

6. Orifice (1) selon la revendication 5, **caractérisé en ce que** la partie de tige (121) comprend une région d'épaulement (125) formée entre la section élargie (122) et la section de pointe (123), dans lequel, dans l'état assemblé de l'élément de fixation (14), le cathéter (2) est serré entre la partie d'épaulement (146) de l'élément de serrage (142) et la région d'épaulement (125) de la partie de tige (121).

7. Orifice (1) selon au moins l'une des revendications précédentes, **caractérisé en ce que** le second élément de contre-verrouillage (105) comprend une face inclinée s'étendant selon un angle incliné par rapport à la direction de fixation (F), le au moins un élément de verrouillage (147) venant en butée contre la face inclinée dans une direction pointant à l'opposé de la direction de fixation (F) dans l'état assemblé de l'élément de fixation (14).

8. Orifice (1) selon au moins l'une des revendications précédentes, **caractérisé en ce que** la partie corps (140) comprend une section d'extrémité (141) au niveau d'une extrémité éloignée par rapport à l'élément de serrage (142), dans lequel la section d'extrémité (141) comprend un élément d'encliquetage (149) qui, dans l'état assemblé de l'élément de fixation (14), est en prise avec un élément de contre-encliquetage (107) formé sur le boîtier (10).

9. Orifice (1) selon au moins l'une des revendications précédentes, **caractérisé en ce qu'**au moins l'un parmi le boîtier (10) et l'élément de fixation (14) comprend un marqueur radio-opaque (15) ayant une structure de marquage perceptible aux rayons X (150).

10. Orifice (1) selon au moins l'une des revendications précédentes, **caractérisé par** un indicateur (16) comprenant une première partie d'indicateur (160) placée sur le boîtier (10) et une seconde partie d'indicateur (161) placée sur l'élément de fixation (14), la première partie d'indicateur (160) et la seconde partie d'indicateur (161) assumant une première position l'une par rapport à l'autre dans l'état pré-assemblé de l'élément de fixation (14) et une seconde position l'une par rapport à l'autre dans l'état assemblé de l'élément de fixation (14), la seconde position indiquant visuellement une attache correcte de l'élément de fixation (14) au boîtier (10) dans l'état assemblé.

11. Orifice (1) selon l'une des revendications précédentes, **caractérisé en ce que** l'orifice (1) présente une forme externe définie par le boîtier (10) et l'élément de fermeture (13) attaché au boîtier (10), dans lequel au moins une pièce d'adaptateur (3, 4) peut être attachée au boîtier (10) pour modifier la forme externe de l'orifice (1), dans lequel l'orifice (1) est opérationnel avec ou sans la au moins une pièce d'adaptateur (3, 4) attachée au boîtier (10).

12. Orifice (1) selon la revendication 11, **caractérisé en ce que** la forme externe est définie par une hauteur maximale (H), une longueur maximale (L) et une largeur maximale, dans lequel la hauteur maximale (H) est mesurée le long d'une direction perpendiculaire à un plan d'extension d'une face inférieure du boîtier (10) opposée à l'ouverture d'accès (109) et la longueur maximale (L) et la largeur maximale sont mesurées dans des directions parallèles au plan d'extension de la face inférieure.
